(19) **Europäisches Patentamt / European Patent Office / Office européen des brevets**

(11) **EP 4 653 006 A1**

(12) # EUROPEAN PATENT APPLICATION

(43) Date of publication:
26.11.2025 Bulletin 2025/48

(21) Application number: 25177703.3

(22) Date of filing: **20.05.2025**

(51) International Patent Classification (IPC):
A61K 36/185 (2006.01)       A61K 31/352 (2006.01)
A61K 35/741 (2015.01)       A61K 35/747 (2015.01)
A61K 36/28 (2006.01)        A61K 36/324 (2006.01)
A61K 36/35 (2006.01)        A61K 36/535 (2006.01)
A61K 36/73 (2006.01)        A61P 1/00 (2006.01)
A61P 19/02 (2006.01)        A61P 29/00 (2006.01)
A61P 11/06 (2006.01)        A61P 11/14 (2006.01)
A61P 37/00 (2006.01)

(52) Cooperative Patent Classification (CPC):
(C-Sets available)
A61K 36/185; A61K 31/352; A61K 35/741;
A61K 35/747; A61K 36/28; A61K 36/324;
A61K 36/35; A61K 36/535; A61K 36/73;
A61P 11/06; A61P 11/14; A61P 29/00; A61P 37/00
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **20.05.2024   IT 202400011356**

(71) Applicant: **Global Pharmacies Partner S.R.L. in
Forma
Abbreviata G.P.P. S.R.L.
20122 Milano (IT)**

(72) Inventors:
• **Torello, Giulio**
  **20122 Milano (IT)**
• **Frati, Francesco**
  **20122 Milano (IT)**
• **Masieri, Simonetta**
  **20122 Milano (IT)**
• **Cavaliere, Carlo**
  **20122 Milano (IT)**

(74) Representative: **Marben S.r.l.**
**Via Larga, 16**
**20122 Milano (IT)**

(54) **NOVEL MIXTURE OF ACTIVE INGREDIENTS, COMPOSITIONS CONTAINING THEM, AND
THEIR USE AGAINST ALLERGY AND/OR INFLAMMATION**

(57)     The present invention relates to a mixture comprising or, alternatively, consisting of boswellia, blackcurrant, helichrysum, perilla, quercetin and, optionally, at least one ingredient selected from the group comprising or, alternatively, consisting of a feverfew extract, apple extract, elderberry extract, one or more strains of probiotic bacteria and/or mixtures thereof, useful in the treatment of allergic and/or inflammatory conditions and symptoms, in particular allergic inflammation, on the one hand, and autoimmune and systemic inflammation, on the other. Furthermore, the invention also refers to pharmaceutical compositions and/or compositions for medical devices (Regulation (EU) 2017/745) and/or nutraceuticals and/or food which comprise such novel mixture and to their use in therapy, in the treatment of allergic and/or inflammatory states and symptoms, in particular allergic inflammation, on the one hand, and autoimmune and systemic inflammation, on the other.

(52) Cooperative Patent Classification (CPC): (Cont.)

C-Sets
**A61K 31/352, A61K 2300/00;**
**A61K 35/741, A61K 2300/00;**
**A61K 35/747, A61K 2300/00;**
**A61K 36/185, A61K 2300/00;**
**A61K 36/28, A61K 2300/00;**
**A61K 36/324, A61K 2300/00;**
**A61K 36/35, A61K 2300/00;**
**A61K 36/535, A61K 2300/00;**
**A61K 36/73, A61K 2300/00**

**Description**

The present invention relates to a mixture comprising or, alternatively, consisting of boswellia, blackcurrant, helichrysum, perilla, quercetin and optionally at least one ingredient selected from the group consisting of or, alternatively, consisting of a feverfew extract, an apple extract an elderberry extract, one or more strains of probiotic bacteria and/or mixtures thereof, useful in the treatment of states and symptoms of an allergic and/or inflammatory nature, in particular allergic inflammation, on the one hand, and autoimmune and systemic inflammation, on the other hand. Furthermore, the invention also relates to pharmaceutical and/or medical device compositions (Regulation (EU) 2017/745) and/or nutraceutical and/or food compositions comprising such novel mixture and to their use in therapy, in the treatment of states and symptoms of an allergic and/or inflammatory nature, in particular allergic inflammation, on the one hand, and autoimmune and systemic inflammation, on the other hand.

The term 'allergy' refers to a class of clinical manifestations caused by mechanisms of individual hypersensitivity to typically harmless substances. The immune system of an allergic individual, in fact, manifests sensitivity to a certain substance that acts as an antigen and this provokes a reaction of varying symptomatology and severity; this hyperreactivity is also called atopy.

The most common clinical manifestations affect both the lower and upper airways with clinical manifestations such as rhino-conjunctivitis or asthma; other important clinical manifestations of the allergic type may affect the skin (dermatitis of an allergic nature) and the gastro-intestinal system, up to and including, in the most severe cases, anaphylaxis and very rarely the dreaded anaphylactic shock, up to and including the death of the affected patient. Allergy is one of the most common chronic diseases in Europe, with an incidence of about 20 per cent. In Europe, the ECRHS (European Community Respiratory Health Survey) has established that the percentage of patients with allergic rhinitis (AR) is between 4% and 32%. More than 40% of patients with AR are asthmatic and more than 80% of asthmatic patients suffer from concomitant rhinitis. National surveys show rhinitis prevalence rates comprised between 5.9% (France) and 29% (UK) with an average of 16% among nations worldwide.

The condition of hyperreactivity of the immune system described, and commonly called allergy, is due to genetic and environmental factors. Diagnosis is generally made by skin reactivity tests (prick tests) or blood tests such as specific IgE assays, while treatment is based, when possible, on prevention of exposure to triggers as well as on use of antihistamine and steroid medications. In the presence of a severe anaphylactic reaction, adrenaline (epinephrine) is used.

The predisposition to allergic reactions derives from polygenic alterations on several chromosomes and it is assumed that the gene sequences involved are also those implicated in asthma; in both cases the symptomatology includes bronchial hyperactivity and overproduction of antibodies such as IgE immunoglobulins in response to the antigenic stimulus.

IgE mediates immediate (or type I) hypersensitivity and is localised on the cell membrane of mast cells (at the tissue level) and basophil granulocytes (at the plasma level); they are antibodies produced upon first contact with the antigen by plasma cells sensitised to the antigen.

The sequence of events that occur following the release of IgE into the plasma circulation includes, in this order:

- adhesion of IgE on the membrane of mast cells, basophilic granulocytes and eosinophilic granulocytes;
- binding between IgE and the allergen to which it is directed (on second contact with the same);
- release of substances contained in the granules of mast cells and basophil granulocytes: histamine, leukotrienes and other mediators that regulate inflammation;
- activation of the sequence of mechanisms characteristic of inflammation and reaction of the body districts involved (dilation of blood vessels, increased vascular permeability, bronchoconstriction, etc.).

This phase of immediate hypersensitivity is followed by a delayed reaction, in which cells involved in the inflammatory mechanism (granulocytes and T lymphocytes) reach the site of contact.

In allergic individuals, the inflammatory response persists and extends to adjacent organs and tissues. In terms of reaction mechanisms, a higher concentration of eosinophilic granulocytes is observed in such individuals, both at the site of the reaction and at blood level. The main actors involved in this process are Th2 lymphocytes (T helper type 2), which release a large quantity of cytokines, responsible for the increased inflammatory response characteristic of allergy (Interleukin 4, Interleukin 13 and Interleukin 5 are the main cytokines involved).

Inflammatory states additional to allergic inflammation are autoimmune inflammatory diseases, such as, for example, rheumatoid arthritis: in this case the lymphocytes most involved are Th1 lymphocytes (T helper type 1). The allergic reaction is clearly an inflammatory reaction and from an immunological point of view is defined as type 2 as it is mainly stimulated by Th2 lymphocytes and consists of 2 phases: 1) the early phase (precocious) occurs a few minutes after contact with the allergen; this phase is mainly caused by degranulation of mast cells and basophils. This phase, if not treated adequately, tends to evolve into 2) late phase; the late phase arises a few hours after contact with the allergen and if left untreated tends to become chronic; the late phase involves the degranulation of eosinophils. An extremely important early phase mediator that causes severe allergic reactions is mast cell tryptase involved in anaphylaxis reactions. The mast cell mediators of the early phase consist of compounds including histamine; the mediators of the late phase are predominantly protein substances, the most important of which is ECP, i.e. the cationic protein of eosinophils.

As mentioned, pharmacological treatments currently used to counteract the inflammatory symptoms related to allergic reaction mainly include antihistamines and corticosteroids. Antihistamines counteract the early phase of the allergic-type inflammatory reaction by a mechanism of interference with histamine receptors present in many cells rich in histamine and other mediators. Such cells are predominantly mast cells and basophils.

Steroids penetrating into cells involved in the late phase of the allergic-type inflammatory reaction negatively modulate (i.e. block or slow down) the production of inflammatory-type substances such as eosinophilic cationic protein and others.

Drugs such as antihistamines and corticosteroids, in order to exert their mechanism of action, may have potential side effects that can also be significant.

With regard to antihistamines, many of the side effects relate to their potential to cross the blood-brain barrier and interact with H1-type receptors present in the central nervous system and produce sedation and sleep; at the peripheral level, mainly in the gastrointestinal tract, interference with H1-type receptors produces unpleasant side effects such as xerostomia, nausea and constipation.

As for the side effects of corticosteroids, most are related to the hormonal effects typical of steroids and include thinning of the skin with stretch marks and haematomas, high blood pressure, increased blood sugar levels, cataracts, swelling of the face (full moon face) and abdomen, thinning of arms and legs, slow wound healing, increased body weight and even obesity.

Therefore, there is a great need to provide a pharmacological and/or nutraceutical alternative or synergistic support preparations with respect to what is known at the state of the art, which are effective in preventing and/or interfering with states and symptoms of an allergic and/or inflammatory nature, and which are free from the side effects, both chemical and hormonal, of the therapies used to date, and which can finally be used for prolonged periods of time by a wide category of subjects.

The Applicant, following intensive research activity, has developed and fine-tuned a novel mixture of active ingredients, and pharmaceutical compositions and/or compositions for medical devices (Regulation (EU) 2017/745) and/or nutraceuticals and/or foodstuffs containing said mixture, which are effective in treating states and symptoms of an allergic and/or inflammatory nature and which are free of side effects, well tolerated and stable over time. The novel mixtures of active ingredients, and the pharmaceutical and/or medical device compositions (Regulation (EU) 2017/745) and/or nutraceutical and/or food compositions containing the same, due to the presence of natural ingredients, do not have chemical or hormonal potential to generate significant side effects and are usable in a broad category of patients also particularly susceptible to the effects of drugs of a chemical nature such as children or the elderly.

The Applicant has found that the novel mixtures, and the compositions of the invention containing the same, do not interfere with the activity of cytochrome P450 (the most important enzyme system of phase I drug metabolism). It follows that, the novel mixtures, and the compositions of the invention containing them, can therefore, not only replace, but also complement, either as a combination therapy or, as a complementary therapy, current drug treatments. The non-interference with cytochrome P450 activity by the mixtures, and compositions of the invention containing them, minimises the risk of drug interactions, in which toxicity may be increased or the therapeutic effects of another drug may be reduced, thus making such mixtures and/or compositions of the invention suitable for use, either as combination therapy or as complementary therapy, as adjuncts to conventionally employed drug treatments. The effect of exposure to increasing concentrations of mixture according to the present invention (Table 2) on the activity of the enzyme CYP2D6, belonging to the cytochrome P450 family, was measured. IC50: concentration at which a 50% reduction in enzyme activity is observed compared to the control (vehicle only).

These objects, and others that will become apparent from the detailed description that follows, are achieved by the novel mixtures of active principles of the present invention, and by the pharmaceutical compositions and/or compositions for medical devices (Regulation (EU) 2017/745) and/or nutraceuticals and/or food products containing the same, thanks to the technical characteristics claimed in the appended claims.

## DETAILED DESCRIPTION OF THE INVENTION

The Applicant has carried out intensive *in-vivo* and *in-vitro* research from which it concluded that the observed effect on Th2-type allergic inflammation could also involve other aspects of the immune response.

The present invention stems from the intuition to use the mixtures and compositions of the present invention in useful preparations to interfere with the inflammatory effects of the Th2-type allergic reaction in the early phase; On the basis of this, in order to evaluate this potential, the antihistamine effect of said mixtures/compositions was tested *in-vivo* by evaluating the main characteristic of an antihistamine drug, i.e. the ability to antagonise mast cell degranulation through interference with the development of the histamine wheal during prick tests.

The Applicant set up an experimental course in a first *in-vivo* step by means of histamine wheal prick tests, and in a second step in order to understand the mechanism of action capable of producing the *in-vivo* effects. The phase of early inflammation was investigated, as well as other potential anti-inflammatory effects not related to the early Th2 phase.

The *in-vivo* study was then also confirmed through an *in-vitro* study using rat leukaemia cells (RBL-2H3 cells), which in modern experimental models have been used as mast cell analogues to evaluate their degranulation in Th2 allergic reactions. In addition to this, on the basis of the excellent results obtained, hypothesising that the complex mechanism of immunological interference of said mixtures/compositions could also involve systemic inflammation and therefore could even interfere in the pathogenetic mechanisms of the autoimmune type, the *in-vitro* study was implemented evaluating the interference of said mixtures/compositions on the complex mechanisms of Th1 inflammation or the interference on the release of an important mediator of autoimmune pathology such as the cytokine TNF alpha.

It is an object of the present invention to provide a mixture (in short, mixture M of the invention) comprising or alternatively consisting of:

(a) a boswellia extract, preferably a resin dry extract, botanical name *Boswellia serrata;* and
(b) a blackcurrant extract, preferably a dry extract of leaves, botanical name *Ribes nigrum;* and
(c) an helichrysum extract, preferably a dry extract of the flowering tops, botanical name *Helichrysum italicum;* and
(d) a perilla extract, preferably a dry extract from seeds/leaves, botanical name *Perilla frutescens*; and
(e) a quercetin; and optionally
(f) at least one ingredient selected from the group comprising or, alternatively, consisting of a feverfew extract,

an apple extract, an elderberry extract, one or more strains of probiotic bacteria, and/or mixtures thereof.

Preferably, said mixture comprises or alternatively consists of:

(a) a boswellia dry extract, preferably a resin dry extract, botanical name *Boswellia serrata*; and
(b) a blackcurrant dry extract, preferably a dry extract of leaves, botanical name *Ribes nigrum*; and
(c) an helichrysum dry extract, preferably an extract of the flowering tops, botanical name *Helichrysum italicum*; and
(d) a perilla dry extract, preferably a seed/leaf extract, botanical name *Perilla frutescens*; and
(e) a quercetin.

An object of the invention is a mixture comprising or alternatively consisting of:

(a) a boswellia dry extract, preferably a resin dry extract, botanical name *Boswellia serrata*; and
(b) a blackcurrant dry extract, preferably a dry extract of leaves, botanical name *Ribes nigrum*; and
(c) an helichrysum dry extract, preferably an extract of the flowering tops, botanical name *Helichrysum italicum*; and
(d) a perilla dry extract, preferably a seed/leaf extract, botanical name *Perilla frutescens*; and
(e) a quercetin;

for use in a preventive or curative method of treatment of an allergic disorder or disease.

The mixture M of the invention as defined above contains (a) a boswellia dry extract (resin, botanical name *Boswellia serrata*).

In the context of the present invention, 'dry extract' means an extract obtained from a plant, or part thereof, according to any technique known to the ones skilled in the art.

*Boswellia serrata* is a tree of the family *Burseraceae* (genus *Boswellia*) that grows in India, North Africa and the Middle East. The resin is extracted by an incision made in the trunk of the tree. Specifically, it is a rubbery oleoresin, consisting of 30-60% resin, 5-10% essential oils soluble in organic solvents, and polysaccharides. The resinous component of *Boswellia serrata* contains monoterpenes, diterpenes, triterpenes, tetracyclic triterpene acids and four main pentacyclic triterpene acids, namely: β-boswellic acid, acetyl-β-boswellic acid, 11-keto-β-boswellic acid and acetyl-11-keto-β-boswellic acid (AKBA).

Preferably, said (a) boswellia dry extract is a dry extract of the resin of *Boswellia serrata,* e.g. obtained by extraction in ethyl acetate with a herb extract ratio of e.g. 10:1.

The extraction ratio, also known as the drug-extract ratio, expresses the amount of raw plant material required to produce a finished extract. For example, a 10:1 ratio means that 10 kg of raw herb is needed to create 1 kg of extract.

Preferably, said (a) boswellia dry extract is a dry extract of the resin of *Boswellia serrata,* having a total boswellic acid titre NLT (not less than) 75%, preferably comprised from 70% to 90%, even more preferably from 75% to 85%. Preferably said (a) boswellia dry extract, preferably a resin dry extract of the of *Boswellia serrata,* comprises one or more of the boswellic acids selected from the group comprising or alternatively consisting of: acetyl-11-keto-β-boswellic acid, alpha-boswellic acid and beta-boswellic acid; more preferably said one or more boswellic acids may be present, in whole or in part, as a phytocomplex, for example a phytocomplex present in a *Boswellia serrata* dry extract.

More preferably, said *Boswellia serrata* dry extract of the resin has a total boswellic acid titre NLT (not less than) 70%, even more preferably NLT 75%.

Preferably, said (a) *boswellia* dry extract is a dry extract of *Boswellia serrata* resin comprising acetyl-11-keto-β-boswellic acid.

More preferably, said (a) *boswellia* dry extract has an acetyl-11-keto-β-boswellic acid titre NLT (not less than) 10%, preferably from 10% to 50%, more preferably from 10% to 20%, as measured by HPLC.

Preferably, said (a) *boswellia* dry extract, preferably a dry extract of *Boswellia serrata* resin, comprises, in addition to acetyl-11-keto-β-boswellic acid, also alpha-boswellic acid. More preferably, said (a) *boswellia* dry extract has an alpha-boswellic acid titre preferably from 1% to 20%, more preferably from 2% to 15%, even more preferably from 2% to 15%, as measured by HPLC.

Preferably, said (a) boswellia dry extract, preferably a dry extract of *Boswellia serrata* resin, comprises, in addition to acetyl-11-keto-β-boswellic acid, beta-boswellic acid. More preferably, said (a) dry extract of boswellia has a beta-boswellic acid titre preferably from 5% to 40%, more preferably from 10% to 30%, even more preferably from 19% to 28%, as measured by HPLC.

Preferably one or more of the above-mentioned boswellic acids (acetyl-11-keto-β-boswellic acid and/or alpha boswellic acid and/or beta-boswellic acid) may be present, in whole or in part, as a phytocomplex; more preferably said phytocomplex may be present in a dry extract of *Boswellia serrata.*

Preferably, said (a) dry extract of *boswellia,* preferably a dry extract of Boswellia serrata resin, comprises or, alternatively, consists of:

- an acetyl-11-keto-β-boswellic acid titre NLT (not less than) 10%, preferably comprised from 10% to 50%, more preferably comprised from 10% to 20%, e.g. 10%, measured by HPLC; and/or
- an alpha-boswellic acid titre preferably comprised from 1% to 20%, more preferably comprised from 2% to15%, even more preferably comprised from 2% to 15%, measured by HPLC; and/or
- a beta-boswellic acid titre preferably comprised from 5% to 40%, more preferably comprised from 10% to 30%, even more preferably comprised from 19% to 28%, measured by HPLC.

More preferably, said (a) boswellia dry extract is a dry extract of *Boswellia serrata* resin, e.g. titrated at 75% total boswellic acid and titrated at 10% acetyl-11-keto-β-boswellic acid. For example, said (a) is the product marketed under the registered name AKBAMAX®.

The mixture M of the invention as defined above contains, in addition to (a) as defined above, (b) a dry extract of blackcurrant (leaves, botanical name *Ribes nigrum*).

Said (b) a dry extract of blackcurrant (leaves, botanical name *Ribes nigrum*) has, for example, CAS No. 68606-81-5.

*Ribes nigrum,* commonly known as blackcurrant, is a plant native to the mountainous areas of Eurasia. The fruits consist of globose black berries rich in seeds that represent a vegetal source of polyunsaturated omega-6 and omega-3 fatty acids present in the form of triglycerides. The buds and leaves contain anthocyanins and flavonoids, including rutin.

Preferably, said (b) blackcurrant dry extract is a dry extract of *Ribes nigrum* leaves, for example obtained by extraction in water/ethanol (70/30 v/v) with an E/D ratio (dry extract compared to the starting drug) of 1:8. Preferably, said (b) blackcurrant dry extract is a dry extract of *Ribes nigrum* leaves, having a rutin titre ≥ 4% and preferably comprised from 4% to 15%, more preferably from 4% to 10%.

Preferably, said (b) blackcurrant dry extract is a dry extract of *Ribes nigrum* leaves, for example titrated at 4% in rutin.

Preferably, said rutin is present, in whole or in part, as a phytocomplex; more preferably said phytocomplex can be present in a dry extract of *Ribes nigrum* leaves.

For example, said (b) is the product with the name "Blackcurrant 4% Rutina/Blackcurrant 4% Rutin" supplied by Nutraceutica s.r.l and comprising, in addition to the dry extract of *Ribes nigrum* leaves, corn maltodextrin as an excipient and maximum 0.5% of anhydrous colloidal silica as an auxiliary substance.

The mixture M of the invention as defined above contains, in addition to (a) and (b) as defined above, also (c) a helichrysum dry extract (flowering tops, botanical name *Helichrysum italicum*).

*Helichrysum italicum* is a perennial shrub of the *Asteraceae* family with fragrant flower heads that grow everywhere in Italy. Said helichrysum dry extract has, for example, CAS No. 90045-56-0.

Inside the flowering tops it contains numerous biologically active compounds such as: essential oils (geraniol, eugenol, neroli, sesquiterpenes, furforol), triterpenes (boswellic acid, ursolic acid), flavonoids (helichrysin, apigenin, quercitrin, narigenin, campferol), caffeic acid, phytosterols, tannins.

Preferably, one or more of the above-mentioned biologically active compounds are present, in whole or in part, as a phytocomplex; more preferably, said phytocomplex may be present in a dry extract of the flowering tops of *Helichrysum*

*italicum.*

More preferably, said (c) dry extract of helichrysum is a dry extract of the flowering tops of *Helichrysum italicum,* for example obtained by extraction in water with an E/D ratio (dry extract compared to the starting drug) of 1:4. For example, said (c) is the product with the name "Elicriso 1/4 / Everlasting 1/4" supplied by Nutraceutica s.r.l and comprising, in addition to the dry extract of helichrysum, corn maltodextrin as an excipient and maximum 0.5% of anhydrous colloidal silica as an auxiliary substance.

The mixture M of the invention as defined above contains, in addition to (a), (b) and (c) as defined above, also (d) a perilla dry extract, preferably a dry extract from seeds or leaves, botanical name *Perilla frutescens. Perilla frutescens* is a herbaceous plant belonging to the *Lamiaceae* family. Preferably, said (d) perilla extract is, for example, a dry extract from seeds or leaves of *Perilla frutescens.* Said extract is for example obtained by extraction in water/ethanol (50/50 v/v) with an E/D ratio (dry extract compared to the starting drug) of 10:1. Preferably, said (d) perilla dry extract has a total polyphenol titre NLT 2.5%, measured by UV, and preferably comprised from 2.5% to 10%, more preferably comprised from 2.5% to 5%, measured by UV.

For example, said perilla dry extract is a dry extract from seeds or leaves of *Perilla frutescens* titrated at 2.5% in total polyphenols, wherein said total polyphenol titre is measured by UV.

Preferably, said polyphenols may be present, in whole or in part, as a phytocomplex; more preferably said phytocomplex may be present in a dry extract of *Perilla frutescens.*

For example, said (d) is the product with the name "Perilla frutescens extract" supplied by Nutrade s.r.l and comprising, in addition to the perilla dry extract, 10% maltodextrin as an excipient.

The mixture M of the invention as defined above contains, in addition to (a), (b), (c) and (d) as defined above, also (e) a quercetin. Quercetin is a flavonoid present in numerous varieties of fruit. Preferably, said quercetin, is granular quercetin with a minimum purity of 95% (measured by HPLC) obtained from *Sophora japonica L.* Preferably, said quercetin is extracted from the flowers of *Sophora japonica L.*

Preferably, said quercetin may be present, in whole or in part, as a phytocomplex; more preferably said phytocomplex may be present in the flowers of *Sophora japonica L.*

For example, said quercetin is the product with the name "Quercetin granular 95%" supplied by Farezis Pharma s.r.l.

The mixture M of the invention as defined above contains, in addition to (a), (b), (c), (d) and (e) as defined above, optionally also (f) at least one ingredient selected from the group comprising or, alternatively, consisting of an extract of feverfew, apple, elderberry, one or more strains of probiotic bacteria and/or mixtures thereof. Preferably, said (f) dry extract of feverfew is, for example, a dry extract of the aerial portion, botanical name *Tanacetum parthenium. Tanacetum parthenium* is a plant of the *Asteraceae* family and contains various flavonoids as well as sesquiterpene compounds such as parthenolide. Preferably, said (f) feverfew extract is a dry extract of the aerial parts of *Tanacetum parthenium,* for example obtained by extraction in water/ethanol (70/30 v/v) with an E/D ratio (dry extract compared to the starting drug) of 1:3-4.

Said (f) dry extract of feverfew has, for example, CAS No. 89997-65-9 and has a parthenolide titre ≥ 0.5% (measured by HPLC).

For example, said (f) dry extract of feverfew is titrated to 0.5% in parthenolides (measured by HPLC).

Preferably, one or more of said parthenolides may be present, in whole or in part, as a phytocomplex; more preferably said phytocomplex may be present in a dry extract of feverfew, preferably a dry extract of the aerial portion of the plant *Tanacetum parthenium.*

For example, said (f) is the product with the name "Partenio 0.5% Partenolidi/Feverfew 0.5% Parthenolides" supplied by Nutraceutica s.r.l comprising, in addition to said extract of feverfew, corn maltodextrin as an excipient and maximum 0.5% anhydrous colloidal silica as an auxiliary substance.

Preferably, said (f) apple extract is, for example, an extract from immature green apples from Central Asia. For example, the apple extract used (Applephenon®) may have a total titre of procyanidins, for example procyanidins B1 and B2, comprised from 50% to 80%, for example comprised from 60% to 70%, and may also contain flavonoids such as, for example, catechin and epicatechin, in an amount comprised from 10% to 15%, by weight.

Preferably, said (f) elderberry extract is, for example, a glyceric extract of elderberry.

Preferably, said (f) elderberry extract (*Sambucus nigra L.*) is, for example, a glyceric extract of elderberry (glycerol 60%-80% and water 20%-40%, extraction ratio 1:2). For example, it may have a CAS No. 84603-58-7. For example, it may have a density (g/ml) comprised of between 1.10 and 1.30 and a pH value comprised of between 4.5 and 6.5.

Preferably, said (f) one or more strains of probiotic bacteria are preferably selected from lactobacilli and/or bifidobacteria, even more preferably at least one lactobacillus and at least one bifidobacterium.

Preferably, said (f) one or more strains of probiotic bacteria are selected from lactobacilli and/or bifidobacteria, even more preferably they are at least one lactobacillus and at least one bifidobacterium. According to an even more preferred aspect of the invention, said probiotics are a *Lactobacillus acidophilus* and a *Bifidobacterium animalis ssp. lactis,* for example *Lactobacillus acidophilus* SGL11 and *Bifidobacterium animalis ssp. lactis* Bi1. Preferably, said lactobacillus can be *Lactobacillus acidophilus* SGL11 and can, for example, be mixed with a corn maltodextrin at a concentration of 10 to 200 billion UFC/g, preferably 50 to 150 billion UFC/g.

Preferably, said bifidobacterium may be *Bifidobacterium animalis ssp. lactis* Bi1 and may, for example, be mixed with maltodextrin or corn starch at a concentration comprised of between $1 \times 10^{11}$ CFU/g to $10 \times 10^{11}$ UFC/g, for example approximately $7 \times 10^{11}$ UFC/g. For example, the strain may be *Bifidobacterium animalis ssp. lactis* Bi1 filed under no. LMG p-17502.

Preferably, said mixture M of the invention comprises or, alternatively, consists of:

(a) a boswellia dry extract, preferably a resin dry extract, botanical name *Boswellia serrata;* and
(b) a blackcurrant dry extract, preferably a dry extract of leaves, botanical name *Ribes nigrum;* and
(c) an helichrysum dry extract, preferably an extract of the flowering tops, botanical name *Helichrysum italicum;* and
(d) a perilla dry extract, preferably an extract from seeds/leaves, botanical name *Perilla frutescens*; and
(e) a quercetin; and, optionally,
(f) at least one ingredient selected from the group comprising or, alternatively, consisting of a feverfew extract, apple, elderberry, one or more strains of probiotic bacteria, and/or mixtures thereof.

Preferably, said (a), boswellia dry extract, is present in said mixture M of the invention in a quantity by weight of 10% to 20%, preferably in a quantity by weight of 12% to 15%, with respect to the total weight of the mixture. Preferably, said (b) blackcurrant dry extract is present in said mixture M of the invention in a quantity by weight of 10% to 20%, preferably in a quantity by weight of 12% to 15%, with respect to the total weight of the mixture. Preferably, said (c) helichrysum dry extract is present in said mixture M of the invention in a quantity by weight of 2% to 10%, preferably in a quantity by weight of 5% to 8%, with respect to the total weight of the mixture. Preferably, said (d) perilla dry extract is present in said mixture M of the invention in a quantity by weight of 20% to 30%, preferably in a quantity by weight of 22% to 28%, with respect to the total weight of the mixture. Preferably, said (e) quercetin, is present in said mixture M of the invention in a quantity by weight of 20% to 30%, preferably in a quantity by weight of 22% to 28%, with respect to the total weight of the mixture.

Preferably, said (f), present in said mixture M, is at least one ingredient selected from the group comprising or, alternatively, consisting of:

- a feverfew dry extract, in a quantity by weight of 10% to 20%, preferably in a quantity by weight of 12% to 15%, with respect to the total weight of the mixture;
- an apple dry extract, in a quantity by weight of 10% to 20%, preferably in a quantity by weight of 12% to 15%, with respect to the total weight of the mixture;
- an elderberry dry extract, in a quantity by weight of 10% to 20%, preferably in a quantity by weight of 12% to 15%, with respect to the total weight of the mixture;
- one or more strains of probiotic bacteria, preferably selected from lactobacilli and/or bifidobacteria, even more preferably at least one lactobacillus and at least one bifidobacterium, in a quantity of 1 billion CFU to 15 billion CFU, for each strain of bacteria present; and/or
- mixtures thereof.

Preferably, the mixture M of the invention comprises or, alternatively, consists of:

(a) a *boswellia* dry extract (i.e. resin) in a quantity of 50 mg to 150 mg, preferably 100 mg; and
(b) a blackcurrant dry extract (i.e. leaves) in a quantity of 50 mg to 150 mg, preferably 100 mg; and
(c) an helichrysum dry extract (i.e. flowering tops) in a quantity of 25 mg to 75 mg, preferably 50 mg; and
(d) a perilla dry extract (i.e. seeds/leaves) in a quantity of 120 mg to 250 mg, preferably 200 mg; and
(e) quercetin in a quantity of 120 mg to 250 mg, preferably 200 mg and, optionally,
(f) a feverfew dry extract (i.e. aerial portion) in a quantity of 50 mg to 150 mg; preferably 100 mg.

For example, a preferred mixture M according to the present invention comprises 100 mg of boswellia dry extract, 100 mg of blackcurrant dry extract, 50 mg of helichrysum dry extract, 200 mg of perilla dry extract, 100 mg of feverfew dry extract, 210 mg of quercetin at 95% (corresponding to 200 mg of quercetin).

Preferably, the mixture M of the invention comprises or, alternatively, consists of:

(a) a boswellia dry extract (i.e. resin) in a quantity of 50 mg to 150 mg, preferably 100 mg; and
(b) a blackcurrant dry extract (i.e. leaves) in a quantity of 50 mg to 150 mg, preferably 100 mg; and
(c) an helichrysum dry extract (i.e. flowering tops) in a quantity of 25 mg to 75 mg, preferably 50 mg; and
(d) a perilla dry extract (i.e. seeds/leaves) in a quantity of 120 mg to 250 mg, preferably 200 mg; and

(e) a quercetin in a quantity of 120 mg to 250 mg, preferably 200 mg; and, optionally,
(f) a feverfew dry extract (i.e. aerial portion) in a quantity of 50 mg to 150 mg; preferably 100 mg; and *Lactobacillus acidophilus* SGL11 and *Bifidobacterium animalis ssp. lactis* Bi1 in a quantity, each, of 1 billion CFU to 15 billion CFU.

For example, a preferred mixture M according to the present invention comprises 100 mg of boswellia dry extract, 100 mg of blackcurrant dry extract, 50 mg of helichrysum dry extract, 200 mg of perilla dry extract, 100 mg of feverfew dry extract, 210 mg of quercetin at 95% (corresponding to 200 mg of quercetin), 1 billion colony forming units (CFU) of *Lactobacillus acidophilus* SGL11 and 1 billion colony forming units (CFU) of *Bifidobacterium animalis ssp. lactis* Bi1.

A further object of the present invention is pharmaceutical compositions and/or compositions for medical devices (Regulation (EU) 2017/745) and/or nutraceutical compositions and/or food compositions comprising (I) a mixture M of the present invention as defined above, i.e. comprising or consisting of (a), (b), (c), (d), (e) and, optionally, (f) (as defined above) and, optionally, (II) at least one additive and/or excipient of pharmaceutical or food grade.

The object of the present invention is also a composition comprising or, alternatively, consisting of (I) a mixture M of the present invention as defined above, or comprising or consisting of (a), (b), (c), (d), (e) and, optionally, (f) (as defined above), and (II) at least one additive and/or excipient of pharmaceutical or food grade, said composition being for use in a method of treatment, preventive or curative, of an allergic disorder or disease.

Preferably, the composition of the invention comprises or, alternatively, consists of:

(I) a mixture M of the invention comprising or, alternatively, consisting of:

(a) a boswellia dry extract (i.e. resin) in a quantity of 50 mg to 150 mg, preferably 100 mg; and
(b) a blackcurrant dry extract (i.e. leaves) in a quantity of 50 mg to 150 mg, preferably 100 mg; and
(c) an helichrysum dry extract (i.e. flowering tops) in a quantity of 25 mg to 75 mg, preferably 50 mg; and
(d) a perilla dry extract (i.e. seed/leaves) in a quantity of 120 mg to 250 mg, preferably 200 mg; and
(e) a quercetin in a quantity of 120 mg to 250 mg, preferably 200 mg; and, optionally, (f) a feverfew dry extract (i.e. aerial portion) in a quantity of 50 mg to 150 mg; preferably 100 mg; and, optionally,

(II) at least one additive and/or excipient of pharmaceutical or food grade.

For example, the composition of the invention comprises or, alternatively, consists of:

(I) a mixture M of the invention comprising or, alternatively, consisting of:

(a) a boswellia dry extract (i.e. resin) 100 mg; and
(b) a blackcurrant dry extract (i.e. leaves) 100 mg; and
(c) an helichrysum dry extract (i.e. flowering tops) 50 mg; and
(d) a perilla dry extract (i.e. seed/leaves) 200 mg; and
(e) a quercetin 200 mg; and, optionally,

(II) at least one additive and/or excipient of pharmaceutical or food grade.

Preferably, the composition of the invention comprises or, alternatively, consists of:

(I) a mixture M of the invention comprising or, alternatively, consisting of:

(a) a boswellia dry extract (i.e. resin) in a quantity of 50 mg to 150 mg, preferably 100 mg; and
(b) a blackcurrant dry extract (i.e. leaves) in a quantity of 50 mg to 150 mg, preferably 100 mg; and
(c) an helichrysum dry extract (i.e. flowering tops) in a quantity of 25 mg to 75 mg, preferably 50 mg; and
(d) a perilla dry extract (i.e. seed/leaves) in a quantity of 120 mg to 250 mg, preferably 200 mg; and
(e) a quercetin in a quantity of 120 mg to 250 mg, preferably 200 mg; and (e) a feverfew dry extract (i.e. aerial portion) in a quantity of 50 mg to 150 mg; preferably 100 mg; and, optionally,
(f) *Lactobacillus acidophilus* SGL11 and *Bifidobacterium animalis* ssp. lactis Bi1 in a quantity, each, of 1 billion CFU to 15 billion CFU; and, optionally,

(II) at least one additive and/or excipient of pharmaceutical or food grade.

The composition of the invention may be a pharmaceutical composition, a composition for a medical device (EU Reg. 2017/745) or a nutraceutical composition or a composition for a food supplement.

Preferably, said compositions of the invention are for oral use.

The compositions of the invention may be formulated preferably for oral administration in a liquid form, such as for example solutions, emulsions, suspensions, sprays, or drops; or in a solid form, such as for example tablets, capsules, granules, powders and equivalent forms known to those skilled in the art, with conventional or modified release.

Preferably, said compositions are in the form of tablets, even more preferably they are in the form of gastro-resistant tablets.

Preferably, the composition according to the invention, preferably in the form of tablets, is administered once or more times a day, for example twice a day. For example, a composition may be administered which comprises or, alternatively, consists of:

(I) a mixture M comprising or, alternatively, consisting of:

(a) a boswellia dry extract (i.e. resin) 100 mg; and
(b) a blackcurrant dry extract (i.e. leaves) 100 mg; and
(c) an helichrysum dry extract (i.e. flowering tops) 50 mg; and
(d) a perilla dry extract (i.e. seed/leaves) 200 mg; and
(e) a quercetin 200 mg; and, optionally,

(II) at least one additive and/or excipient of pharmaceutical or food grade.

Preferably, by means of the composition of the present invention, a daily dose of a boswellia dry extract equal to 200 mg can be administered, for example, by means of one administration, or by means of two administrations. Preferably, by means of the composition of the present invention, a daily dose of a blackcurrant dry extract equal to 200 mg can be administered, for example, by means of one administration, or by means of two administrations. Preferably, through the composition of the present invention, a daily dose of an helichrysum dry extract equal to 100 mg can be administered, for example, through one administration, or through two administrations.

Preferably, through the composition of the present invention, a daily dose of perilla dry extract equal to 400 mg can be administered, for example, through one administration, or through two administrations.

Preferably, through the composition of the present invention, a daily dose of quercetin equal to 400 mg can be administered, for example, through one administration, or through two administrations.

Preferably, said compositions are in liquid form in drops for administration preferably to subjects of pediatric age, for example, from 3 years to 18 years.

The methods of production of these forms are known to the ones skilled in the art, who can select, for their preparation, any technique, equipment and excipient suitable for the purpose.

The compositions of the invention as defined above comprise (II) at least one additive and/or excipient of pharmaceutical or food grade, such as a substance without therapeutic activity suitable for pharmaceutical or food use, such as, for example but not limited to, diluents, glidants, disintegrants, solubilizers, anti-caking agents, coating agents, plasticizers, sweeteners, flavorings, colorants, surfactants, antimicrobials, antioxidants, preservatives, buffers to stabilize the pH, acidifiers, and all auxiliary substances known to the ones skilled in the art.

Preferably, said (II), at least one additive and/or excipient of pharmaceutical or food grade, is selected from, at least one, preferably all, of the following excipients: microcrystalline cellulose, magnesium salts of fatty acids, silicon dioxide, shellac, hydroxypropyl methylcellulose, talc, polyethylene glycol, red iron oxide, calcium carbonate.

Said mixture M of the invention, or compositions comprising it, are for use in therapy, preferably for use in a method of treatment, preventive or curative, of an allergic disorder or disease.

A further object of the present invention is a mixture M of the invention or a composition of the invention comprising said mixture M, as defined above, for use in therapy, in particular in a method of treatment, preventive and/or curative and/or symptomatic, of states and symptoms of an allergic and/or inflammatory nature, in particular of allergic inflammation, on the one hand, and autoimmune and systemic inflammation, on the other. Preferably, said mixture M of the invention, or compositions comprising it, are for use in therapy, in particular in a method of treatment, preventive and/or curative and/or symptomatic, of states and symptoms of an allergic and/or inflammatory nature, in particular of allergic inflammation, on the one hand, and autoimmune and systemic inflammation, on the other, for example, of respiratory disorders of an allergic nature such as rhinorrhea, dry cough, angioedema of the upper airways, bronchospasm.

Preferably, said mixture M of the invention, or compositions comprising it, are for use in therapy, in particular in a method of treatment, preventive and/or curative and/or symptomatic, of states and symptoms of an allergic and/or inflammatory nature, in particular of allergic inflammation, on the one hand, and autoimmune and systemic inflammation, on the other, for example, in the case of rheumatoid arthritis.

The object of the present invention are methods for the preventive and/or curative and/or symptomatic treatment of allergic and/or inflammatory conditions and symptoms, in particular allergic inflammation, on the one hand, and autoimmune and systemic inflammation, on the other, for example, respiratory disorders of an allergic nature such as rhinorrhea, dry cough, angioedema of the upper airways, bronchospasm, by administering, preferably orally, to a subject in need, an effective quantity of a mixture M of the invention or of a composition comprising it as defined above.

The object of the present invention are methods for the preventive and/or curative and/or symptomatic treatment of allergic and/or inflammatory conditions and symptoms, in particular allergic inflammation, on the one hand, and autoimmune and systemic inflammation, on the other, for example, in the case of rheumatoid arthritis, by administering, preferably orally, to a subject in need, an effective quantity of a mixture M of the invention or of a composition comprising it as defined above.

The mixture M of the invention or the compositions comprising it can be administered as a single treatment and/or in combination with other compositions or therapies (i.e., with adjuvant action) useful in the preventive and/or curative and/or symptomatic treatment of allergic and/or inflammatory states and symptoms.

Advantageously, the mixtures and/or compositions of the present invention, in fact, do not interfere with the activity of cytochrome P450 and, therefore, can also be used, both as a combination therapy and as a complementary therapy, as adjuvants to conventionally employed pharmacological treatments, wherein the risk of pharmacological interactions in which the toxicity is increased or the therapeutic effects of another drug are reduced, is minimal.

In the context of the present invention, combination therapy means a simultaneous or sequential administration, preferably oral, of an effective quantity of a mixture M of the invention, or of a composition comprising it, and of a further drug conventionally used for said allergic and/or inflammatory states and symptoms. Simultaneous administration means that said effective quantity of mixture M of the invention, or of a composition comprising it, and said effective quantity of a further drug conventionally used are administered to the subject in need at the same time (either in the form of a single dosage form or as two distinct dosage forms taken at the same time). Sequential administration means that said effective quantity of mixture M of the invention, or of a composition comprising it, and said effective quantity of a further drug conventionally used are administered to the subject in need at very close moments in time, i.e. one after the other within a few seconds or within a few minutes, for example 1 minute, 2 minutes, 5 minutes, 10 minutes (typically as two distinct dosage forms taken in rapid sequence).

In the context of the present invention, complementary therapy means a deferred administration in time, preferably oral, of an effective quantity of a mixture M of the invention, or of a composition comprising it, and of a further drug conventionally used for said states and symptoms of an allergic and/or inflammatory nature. By time-delayed administration it is meant that said effective quantity of mixture M of the invention, or of a composition comprising it, and said effective quantity of a further conventionally used drug are administered to the subject in need at times widely spaced apart in time, according to a therapeutic scheme prescribed by the physician as needed. For example, the administration of a mixture M, or of a composition comprising it, and of a further conventionally used drug (typically as two distinct dosage forms taken at times widely spaced apart in time) occurs at a distance of hours, for example 2 hours, 3 hours, 4 hours, 8 hours, 12 hours, or at a distance of days, for example 1 day, 2 days, 7 days, or at a distance of weeks, for example 1 week, 2 weeks, 3 weeks, 4 weeks, one from the other. The definition of complementary therapy according to the present invention also includes therapeutic schemes that involve the intake of only the mixture M, or of the composition comprising it, (or of only the additional drug conventionally used) for a certain period of time, for example 4 days, 7 days, 10 days, two weeks, 4 weeks, followed by the intake of only the additional drug conventionally used (or of only the mixture M of the invention, or of the composition of the invention comprising said mixture M) for a certain subsequent period of time, for example 4 days, 7 days, 10 days, two weeks, 4 weeks; also as a repeated therapeutic scheme with variable single-administration time intervals.

Therefore, a further object of the present invention is a mixture M of the invention or a composition of the invention comprising said mixture M, as defined above, for use in a combination therapy or in a complementary therapy, in particular in a method of treatment, preventive and/or curative and/or symptomatic, of states and symptoms of an allergic and/or inflammatory nature.

Preferably, said mixture M of the invention, or the compositions comprising it, are for use in a combination therapy or in a complementary therapy, in particular in a method of treatment, preventive and/or curative and/or symptomatic, of states and symptoms of an allergic and/or inflammatory nature, in particular of disorders affecting the respiratory system of an allergic nature such as rhinorrhea, dry cough, angioedema of the upper airways, bronchospasm.

Preferably, said mixture M of the invention, or the compositions comprising it, are for use in a combination therapy or in a complementary therapy, in particular in a method of treatment, preventive and/or curative and/or symptomatic, of states and symptoms of an allergic and/or inflammatory nature, in particular of allergic inflammation and autoimmune inflammation, for example in the case of rheumatoid arthritis.

The object of the present invention is a method for the preventive and/or curative and/or symptomatic treatment of allergic and/or inflammatory states and symptoms, in particular of allergic respiratory disorders such as rhinorrhea, dry cough, angioedema of the upper airways, bronchospasm, by means of the simultaneous, sequential and/or deferred administration, preferably orally, to a subject in need, of an effective quantity of a mixture M of the invention or of a composition comprising it as defined above and of a further drug conventionally used for said allergic and/or inflammatory states and symptoms.

The object of the present invention are methods for the preventive and/or curative and/or symptomatic treatment of allergic and/or inflammatory states and symptoms, in particular allergic inflammation and autoimmune inflammation, for example in the case of rheumatoid arthritis, by means of the simultaneous, sequential and/or deferred administration, preferably orally, to a subject in need, of an effective quantity of a mixture M of the invention or of a composition comprising it as defined above and of a further drug conventionally used for said allergic and/or inflammatory states and symptoms.

The appropriate dosage of the M mixture of the invention, or of a composition comprising it, whether as a single treatment or as a combination therapy or complementary therapy, will depend, for example, on the body weight, the condition to be treated/prevented, the severity and of course the condition, whether the M/composition mixture is administered for preventive or therapeutic purposes, any previous therapy, the patient's medical history and response to the M/composition mixture and is determined at the discretion of the treating physician.

For example, the composition of the invention is appropriately administered to the subject at one time or distributed in a series of treatments. Preferably, the composition of the invention is administered once or twice daily in a single dose.

The term "subjects" in the context of the present invention refers to human subjects for human use or animal subjects for veterinary use (e.g., companion animals such as dogs or cats or other mammals).

Preferably, the mixtures and compositions of the invention are for use in methods of treating human subjects.

In the context of this invention, "treatment method" means an intervention, comprising the administration of a substance, or mixture of substances or combination thereof, having as its purpose the elimination, reduction/diminution or prevention of a pathology or disease and its symptoms or disorders.

The term "medical device" in the context of this invention is used in the meaning according to the Italian Legislative Decree of 24 February 1997, n. 46 (or according to the new Medical Devices Regulation (EU) 2023/607 (MDR)), i.e. it indicates a substance or another product, used alone or in combination, intended by the manufacturer to be used in humans for the purpose of diagnosis, prevention, control, therapy or alleviation of a disease, which product does not exert its principal action, in or on the human body, for which it is intended, by pharmacological or immunological means or by metabolic process, but whose function can be assisted by such means.

Unless otherwise specified, the indication that a mixture or composition "comprises" one or more components or substances means that other components or substances may be present, in addition to the one or those specifically indicated. For example, if desired or necessary, to said substances (a), (b), (c), (d), (e), and, optionally, (f), other extracts or active substances may also be added that contribute to the effectiveness of the mixture or composition. Unless otherwise specified, the expression mixture or composition comprising a component in a quantity "comprised in a range from x to y" or "from x to y" means that said component may be present in the mixture or composition in all the quantities present in said range, even if not explicitly stated, including the extremes of the range.

The mixture or composition of the present invention is prepared by mixing the single components that constitute it or according to any other method considered valid by the ones skilled in the art. According to a preferred aspect of the invention, at the end of the mixing, a compression process and subsequent coating are carried out, preferably a gastro-resistant coating.

It is an object of the present invention a composition in solid form for oral use, preferably a tablet, which comprises a mixture of a), b), c) and e) and, optionally, at least one f), said composition being for use in therapy in a method of treatment, preventive and/or curative and/or symptomatic, anti-allergic of a disorder or disease affecting the respiratory system of an allergic nature; or said mixture being for use in therapy in a method of treatment, preventive and/or curative and/or symptomatic, of an inflammatory disease of the autoimmune type or intestinal inflammatory or allergic pathology.

Preferably, the composition of the invention in solid form for oral use in the form of a tablet such as, for example, an oblong gastro-resistant tablet of the type in Table 1:

Table 1

| Component | Quantity (mg) | Specifications |
| --- | --- | --- |
| Dry extract gum-resin 75% boswellic acids, 10% keto-boswellic acid | 100 | Boswellic acids 75 mg; ketoboswellic acid 10 mg |
| Blackcurrant dry extract from leaves 4% rutin | 100 | Rutin 4 mg |
| Helichrysum dry extract from flowering tops | 50 | |
| Perilla dry extract from seed 2.5% polyphenols | 200 | Polyphenols 5 mg |
| Granuled quercetin (95%) | 210 | Quercetin 200 mg |
| Feverfew dry extract 0.5% parthenolides | 100 | Parthenolides 0.5 mg |

(continued)

| Component | Quantity (mg) | Specifications |
|---|---|---|
| *Lactobacillus acidophilus* SGL11 150x10$^9$ per gram | 13.3 | 1 billion CFU of live cells |
| *Bifidobacterium animalis ssp. lactis* Bi1 200x10$^9$ per gram | 10 | 1 billion CFU of live cells |
| Microcrystalline cellulose | 176.7 | |
| Silicon dioxide | 20 | |
| Magnesium salts of fatty acids | 20 | |
| Total | 1,000 | |

The object of the present invention is a composition in solid powder form for oral use, preferably a powder for a sachet, for example a bipartite sachet, which comprises a mixture of a), b), c) and e) and, optionally, at least one f), said composition being for use in therapy in a method of treatment, preventive and/or curative and/or symptomatic, anti-allergic of a disorder or disease affecting the respiratory system of an allergic nature; or said mixture being for use in therapy in a method of treatment, preventive and/or curative and/or symptomatic, of an inflammatory disease of the autoimmune type or intestinal inflammatory or allergic pathology.

Preferably, the composition of the invention is in solid powder form for oral use, preferably in the form of a powder contained in a bipartite sachet formed by two chambers, a first chamber A and a second chamber B, adjacent to each other. The two chambers A and B are made of a material known for this type of sachet. For example, the two chambers A and B are adjacent to each other. Each of the two chambers A and B defines a closed, but openable volume, equal or different from each other, depending on the composition they must contain. Preferably, chamber A of said divided sachet may contain: *Lactobacillus acidophilus* SGL11 150x10$^9$ per gram, equal to 33.33 mg/sachet (1x10$^9$ UFC); *Bifidobacterium animalis ssp. lactis* Bi1 200x10$^9$ per gram, equal to 25 mg/sachet (1x10$^9$ UFC); and chamber B of said divided sachet may contain: Dry extract of gum-resin 75% boswellic acids, 10% keto-boswellic acid, 100 mg/sachet; Dry extract of blackcurrant leaves 4% rutin, 100 mg/sachet; Dry extract of helichrysum from flowering tops, 50 mg/sachet; perilla dry extract from seed 2.5% polyphenols, 200 mg/sachet; Granular quercetin (95%), 250 mg/sachet; Apple extract 50 mg/sachet.

The object of the present invention is a composition in liquid form for oral use, preferably in liquid form in drops, which comprises a mixture of a), b), c) and e) and, optionally, at least one f), said composition being for use in therapy in a method of treatment, preventive and/or curative and/or symptomatic, anti-allergic of a disorder or disease affecting the respiratory system of an allergic nature; or said mixture being for use in therapy in a method of treatment, preventive and/or curative and/or symptomatic, of an inflammatory disease of an autoimmune type or intestinal inflammatory or allergic pathology.

Preferably, the composition of the invention can be in liquid form in drops for administration also to subjects of pediatric age, for example it can be of the type (20 ml or 30 ml or 40 ml or 50 ml): Elderberry glyceric extract: from 10 mg/0.5 ml to 30 mg/0.5 ml, for example 20 mg/0.5 ml; Blackcurrant glyceric extract: from 5 mg/0.5 ml to 15 mg/0.5 ml, for example 10 mg/0.5 ml; Perilla dry extract from leaves 5% polyphenols: from 1 mg/0.5 ml to 5 mg/0.5 ml, for example 3 mg/0.5 ml; Quercetin 93% from Sophora Japonica: from 1 mg/0.5 ml to 5 mg/0.5 ml, for example 3 mg/0.5 ml; Helichrysum dry extract (flowering herb) 1:4: from 1 mg/0.5 ml to 5 mg/0.5 ml, for example 2.5 mg/0.5 ml; Boswellia dry extract titrated at 65% in boswellic acids: from 1 mg/0.5 ml to 5 ml/0.5 ml, for example 2.5 mg/0.5 ml; and water, sodium benzoate, potassium sorbate, glycerin, fructose, flavouring and lactic acid. The daily dosage can be 0.5-1.5 ml: 10-15 drops, for example for subjects aged between 3 and 12 years.

**EXPERIMENTAL PART**

*In-vivo* study

The efficacy of a mixture M according to the present invention was also tested in an *in-vivo* study conducted on nine volunteer subjects.

The antihistamine efficacy of a mixture M, and a composition containing the same, was tested *in-vivo*, by administering it before the histamine prick test in 9 subjects, 7 males and 2 females, aged from 26 to 63 years (average age approximately 45 years) for a number of times equal to 1 time per day for 30 days.

We repeated this histamine prick test after 15 and after 30 days of treatment with said mixture M at a dose of 1 tablet.

In particular, the 9 subjects (7 males and 2 females) were enrolled on a voluntary basis and subjected to a prick test, i.e. a type of skin test normally used in diagnostics to monitor allergic reactions to different types of substances. When used in diagnostics, this test requires that modest quantities of the substance suspected of causing the allergic reaction are applied to the patient. The application usually occurs on the inner forearm, facilitating its penetration into the skin through the prick test method with a special disposable lancet. If a skin eruption occurs at the application site, in the form of a rash, wheals or red patches, the patient is most likely positive for that type of allergen and a diagnosis can be made.

Histamine is used as a positive control, which can generate, in all subjects, allergic and non-allergic, a positive skin reaction (histamine wheal).

The antiallergic efficacy of a mixture M according to the present invention was evaluated by subjecting subjects, who were not undergoing therapy with antihistamine drugs, to a prick test with histamine and inert solution as a negative control at time zero (T0) and after 15 (T1) and 30 (T2) days of therapy with the mixture of the invention, reported in the following Table 2, taken in the form of a gastro-resistant tablet 1 or 2 times a day.

The evaluation was made by measuring the size of the wheal that developed following a prick test with histamine at the three times considered, and subsequent comparison between the size of the wheal that had formed at T0 and the subsequent ones. The results were statistically analyzed using the Wilcoxon and Mann-Whitney tests. The interference (decrease) in the size of the histamine wheal is correlated with the antihistamine effect and, therefore, with an antiallergic effect.

The tested mixture is reported in the following Table 2 and was taken in the form of a gastro-resistant tablet one (4 subjects) or two (5 subjects) times a day, after the main meals.

Table 2: qualitative/quantitative composition of the mixture according to the present invention tested in the *in vivo* study

| Component | Quantity (mg) | Specification |
| --- | --- | --- |
| Gum-resin dry extract 75% boswellic acids, 10% ketoboswellic acid | 100 | Boswellic acids 75 mg; ketoboswellic acid 10 mg |
| Blackcurrant dry extract from leaves 4% rutin | 100 | Rutin 4 mg |
| Helichrysum dry extract from flowering tops | 50 | |
| Perilla dry extract from seed 2.5% polyphenols | 200 | Polyphenols 5 mg |
| Granuled quercetin (95%) | 210 | Quercetin 200 mg |
| Feverfew dry extract 0.5% parthenolides | 100 | Parthenolides 0.5 mg |
| *Lactobacillus acidophilus* SGL11 $150 \times 10^9$ UFC | 13.3 | 1 billion CFU of live cells |
| *Bifidobacterium animalis ssp. lactis* Bi1 $200 \times 10^9$ UFC | 10 | 1 billion CFU of live cells |
| Microcrystalline cellulose | 176.7 | |
| Silicon dioxide | 20 | |
| Magnesium salts of fatty acids | 20 | |
| Total | 1,000 | |

The measurement of the size of the histamine wheals at the selected times and the application of the statistical analysis according to the Wilcoxon test, allowed to highlight a significant difference in the size of the histamine wheal both between the times T0 and T1 ($p = 0.1$; $z = -2.25$) and between the times T0 and T2 ($p = 0.4$; $z = -2.02$). The verification regarding the existence of a significantly different result between the subjects who took the mixture according to the invention 1 or 2 times a day was done through the Mann-Whitney statistical test.

In conclusion, it can be stated that the treatment with the mixture according to the present invention (Table 2) determined, already after 15 days (T1), a notable decrease in the size of the histamine wheal with statistically significant differences compared to the time of its induced onset (T0). These differences compared to T0 are maintained even in the case of 30 days of therapy (T2). Furthermore, the comparison between the results obtained with a single (1 tablet per day) or repeated (two tablets per day) administration treatment, have highlighted how even a single administration is sufficient to guarantee the antihistamine effect and that this effect is achieved in just 15 days (T1).

These *in-vivo* experimental results confirm the interference effect of the mixture according to the present invention with respect to the mediators released during the allergic reaction (prick test). In particular, the rapid action times and how the administration of a single dose is sufficient and the possibility of administration for prolonged periods are to be noted.

*In vitro* study

To evaluate the efficacy of a mixture M according to the invention, the following *in vitro* study was conducted.

The antiallergic activity of the tested sample of mixture M was evaluated by measuring its ability to prevent degranulation processes in a rat leukemia cell line (RBL-2H3), widely used as a model simulating mast cells in immunological and allergological studies.

The anti-inflammatory action was instead evaluated, always in the same cell line, by measuring the ability of the sample to inhibit the release of tumor necrosis factor alpha (TNF$\alpha$).

A preliminary cell viability test was first performed, in order to identify the concentrations of the product on which to conduct the subsequent tests. Based on the results obtained, it was decided to perform the efficacy evaluations using the concentrations of 0.02 and 0.01 mg/mL.

The RBL-2H3 cells were first sensitized and then stimulated to induce degranulation. A series of cells did not undergo any type of treatment (NS), a series of cells was sensitized/stimulated but was not treated (NC, negative control), while a series of cells was sensitized/stimulated and treated with the test sample at the concentrations selected based on the results of the preliminary cytotoxicity test.

In the cells treated with the tested sample at the tested concentrations of 0.02 and 0.01 mg/ml, a significant reduction in degranulation, by 35.1% and 12.7%, respectively, and in TNF$\alpha$ release, by 13.0% and 11.9%, respectively, was observed compared to the NC control cells.

MATERIALS, METHODS AND TESTS PERFORMED

Cell cultures

The test was conducted on RBL-2H3 cells grown in DMEM (Dulbecco's modified Eagle medium) containing 10% fetal bovine serum (FBS) and 1% antibiotics (penicillin and streptomycin) and incubated in standard culture conditions (37°C, 5% $CO_2$). RBL-2H3 cells are widely used in allergy and immunology research, as, like mast cells, they express FC$\epsilon$RI and, following cross-linking between IgE bound to FC$\epsilon$RI and allergens, they undergo degranulation. In this way, therefore, they simulate the behavior of mast cells and basophils in their response to an immunological stimulus.

Sample preparation

The mixture M according to the invention used for the present *in vitro* study has the following qualitative/quantitative composition of active ingredients equal to 15 tablets because the laboratory needed to have approximately 1/15 grams of powder for the operation i.e. solubility tests and serial dilutions.

The mixture M for 15 tablets is reported in Table 3.

Table 3

| | |
|---|---|
| AKBAMAX® D.E. gum-resin<br>75% Boswellic Acids<br>10% Keto-boswellic acid | 1,500 mg |
| BLACKCURRANT 4% RUTIN leaves D.E. | 1,500 mg |
| GRANULAR QUERCETIN 95% | 3,150 mg |
| HELICHRYSUM<br>¼ flowering tops D.E. | 750 mg |
| PERILLA 2.5% POLYPHENOLS seed D.E. | 3,000 mg |
| FEVERFEW 0.5% PARTHENOLIDES flowering tops D.E. | 1,500 mg |
| L.ACIDOPHILUS SGL11 150 billion/gram | 199.5 mg |
| B.ANIMALIS ssp. LACTIS Bi1 200 billion/gram | 150 mg |

The mixture M under examination was found to be soluble in DMSO at a concentration of 2.0 mg/mL. To prevent the solvent from interfering with the test result, the starting solution was diluted 100-fold in complete culture medium or in the buffer used for the test. Therefore, the cells were treated with the sample thus prepared starting from the concentration of 0.02 mg/mL and subsequent 1:2 dilutions (always in culture medium/buffer).

Evaluation of cell viability

Before the anti-allergy and anti-inflammatory test, a cell viability assay was conducted in order to select the concentrations on which to perform the subsequent analyses. Cell viability was assessed by MTT [3-(4,5-dimethylthiazol-2-yl)-2,5-diphenyltetrazolium bromide] test, a yellow tetrazole compound that is reduced by the cells to purple formazan. This conversion is caused by NADPH or NADH produced by dehydrogenases present in metabolically active cells. Cells were treated with MTT (1 mg/ml) and incubated for 3 h at standard conditions. At the end of this period, the MTT solution was discarded and 100 $\mu$l of isopropanol was added to each well to dissolve the formazan crystals that had formed. The absorbance (optical density, OD) was determined by reading the spectrophotometer at a wavelength of 570 nm. The absorbance (optical density, OD), measured spectrophotometrically at 570 nm, is proportional to cell viability. The percentages of cell viability were calculated based on the OD values measured at 570 nm and considering the OD of the negative control (NC, untreated cells) as 100% viability. TS = cells treated with the tested sample of the mixture according to the invention, prepared as previously described.

Based on the results obtained, to perform the subsequent tests (evaluation of degranulation and dosage of TFN$\alpha$) it was therefore decided to use the sample concentrations equal to 0.02 mg/mL; and 0.01 mg/mL.

Evaluation of degranulation (evaluation of antiallergic activity)

Following degranulation, the release of $\beta$-hexosaminidase is positively correlated with the release of histamine, which is one of the most abundant mediators in mast cell granules. Therefore, the release of $\beta$-hexosaminidase is commonly used as a marker of mast cell degranulation.

RBL-2H3 cells were sensitized with anti-dinitrophenyl immunoglobulin E (anti-DNP IgE, 200 ng/ml overnight) and then stimulated with human dinitrophenyl albumin (DNP-HSA, 100 ng/ml for 60 minutes). One set of cells was neither sensitized nor stimulated (NS); a second set was sensitized and stimulated but was not treated (NC, negative control); a third set of cells was pretreated with the test sample and then sensitized and stimulated. Dehydrocostolactone 20 $\mu$M, a natural sesquiterpene lactone contained in *Saussurea Costus,* and with known anti-inflammatory and anti-allergic activities, was used as a positive control (PC).

The medium was then recovered and separated from the cells and incubated with 4-nitrophenyl-N-acetyl-$\beta$-D-glucosamide. The reaction was stopped with carbonate buffer and the absorbance was measured by spectrophotometric reading at 405 nm. Degranulation was calculated as the percentage of $\beta$-hexosaminidase relative to non-sensitized (NS) cells as follows:

$$Degranulation\ (\%) = \frac{OD_X}{OD_{NS}} \cdot 100$$

The measured absorbance (optical density, OD) is directly proportional to the amount of $\beta$-hexosaminidase present in the analyzed fluids, which in turn correlates positively with the levels of histamine released during the degranulation phase and is therefore an indicative marker of the degranulation process. The percentages were calculated based on the measured absorbance values and represent the percentage of $\beta$-hexosaminidase compared to non-sensitized and non-stimulated cells (NS). The values are expressed as means $\pm$ standard deviation. Statistical processing of the data was performed by Student's t-test. Values of $p<0.05$ were considered significant. NC = negative control (sensitized, stimulated and untreated cells); PC = positive control (dehydrocostolactone 20 $\mu$M); **$p<0.01$ vs NC.

Table 4: results relating to the evaluation of degranulation in cell cultures treated with the sample at two different concentrations (TS) compared to controls (negative, NC and positive, PC) and to non-sensitized and non-stimulated cells (NS).

| | NC | TS, sample concentration (mg/ml) | | PC |
| --- | --- | --- | --- | --- |
| | | *0.02* | *0.01* | |
| $\beta$-hexosaminidase (% vs NS) | 1,274.6 | 827.0 | 1,112.4 | 244.8 |
| protection (% vs NC) | - | 35.1 | 12.7 | 80.8 |

The results reported in Table 4 demonstrate the ability of the mixture according to the present invention to significantly reduce degranulation at the tested concentrations of 0.02 and 0.01 mg/ml (reduction of 35.1% and 12.1% respectively compared to untreated cells, indicated as protection%). This result is indicative of an antiallergic activity.

TNF$\alpha$ dosage (evaluation of anti-inflammatory activity)

The supernatant recovered from the cells was also used for the dosage of TNF$\alpha$ performed by ELISA (Enzyme-Linked Immunosorbent Assay), a very widespread biochemical technique used for the quantification of proteins and peptides. In an ELISA assay, a specific antigen for TNF$\alpha$ is immobilized on a solid surface (the bottom of a well) to which the assay medium is added. Detection is done by means of a biotinylated secondary antibody and then reacted with streptavidin-HRP. The colorimetric reaction is proportional to the amount of TNF$\alpha$ present. The results are read by spectrophotometer at 450 nm. The values obtained were then interpolated into a standard curve of TNF$\alpha$.

The absorbance (optical density, OD) measured at 450 nm is directly proportional to the amount of TNF$\alpha$ produced by the tissues. To calculate the concentrations of TNF$\alpha$, the OD values obtained were interpolated into a standard curve of TNF$\alpha$. The percentages were calculated with respect to the non-sensitized and non-stimulated cells (NS). The values are expressed as means $\pm$ standard deviation. Statistical processing of the data was performed by Student's t-test. Values of $p<0.05$ are considered significant. NC = negative control (sensitized, stimulated and untreated cells); PC = positive control (dehydrocostolactone 20 $\mu$M); $**p<0.01$ vs NC.

Table 5: results relating to the evaluation of TNF$\alpha$ dosage in cell cultures treated with the sample at two different concentrations (TS) compared to controls (negative, NC and positive, PC) and to non-sensitized and non-stimulated cells (NS).

| | NC | TS, sample concentration (mg/ml) | | PC |
| --- | --- | --- | --- | --- |
| | | 0.02 | 0.01 | |
| TNF$\alpha$ (pg/ml) | 248.8 | 216.5 | 219.2 | 200.3 |
| TNF$\alpha$ | 227.8 | 198.2 | 200.7 | 183.4 |
| (% vs NS) | | | | |
| protection (% vs NC) | - | 13.0 | 11.9 | 19.5 |

The results reported in Table 5 demonstrate the ability of the mixture according to the present invention to significantly reduce the production of TNF$\alpha$ at the tested concentrations of 0.02 and 0.01 mg/ml (reduction, or protective action%, of 13.0% and 11.9%, respectively, compared to untreated cells).

In light of the experimental evidence obtained from both *in vitro* and *in vivo* studies, the mixture of the invention has proven to be able to exert an antihistaminic and anti-inflammatory effect. The action is exerted rapidly, with the possibility of administration for prolonged periods. Furthermore, by virtue of the fact that the mixture of the invention contains natural ingredients, it does not present significant adverse effects, is free of side effects and has high tolerability, and can be administered to a wide category of subjects.

Safety of the mixture according to the invention - *In vitro* study

In order to further evaluate the safety of the mixture M according to the present invention, the inhibitory effect of the previous mixture of the present invention (as reported in Table 2) on the enzymatic activity of a subtype of Cytochrome P450, in particular CYP2D6, was measured.

The potential inhibitory effect is evaluated by means of a luminometric test, i.e. a test that quantifies the light intensity emitted by a given species; in the case in question, when an increase in light intensity is measured, it indicates a greater presence of metabolic product and therefore a lower inhibition of the subtype of Cytochrome P450 (CYP450). The test used involves the use of a membrane containing human recombinant CYP2D6 and a membrane without cytochrome used as a negative control. To perform the test, a substrate is added which, by reacting with the cytochrome P450 (CYP2D6 subtype) generates a product, which, by reacting with luciferase, emits light. The amount of light emitted will therefore be proportional to the amount of product obtained and consequently to the percentage of cytochrome that has not undergone any inhibition.

Cytochrome P450 (CYP450) is intended to indicate a family of enzymes responsible for oxidative metabolism in the body. These enzymes play a primary role in the metabolism of many drugs and are implicated in most phase 1 metabolic processes in the liver.

The induction and inhibition of these enzymes can be modulated by multiple factors, both genetic (sex, race, age, etc.) and exogenous (nutrition, lifestyle, drugs, etc.). Alterations to this system can lead to consequences on the absorption, metabolism, half-life and bioavailability of the active ingredients. For this reason, it is important to measure the impact that the mixture of the present invention could have on this family of receptors. In particular, the measured inhibition effect must be as low as possible, so as to demonstrate the non-interference of the mixture according to the present invention with the metabolism of other drugs or nutrients.

The inhibition effects of the mixture M according to the present invention (as reported in Table 2), are compared with those deriving from a compound that is a known and selective inhibitor for the considered receptor isoform CTP2D6.

The scientific literature divides inhibitors of CYP450, and its isoforms, into potent, moderate or weak inhibitors, on the basis of measured IC50 values (50% inhibitory concentration of enzyme activity). In particular, a molecule is considered a potent inhibitor if it shows an IC50 value $\leq$ 10 $\mu$g/mL, a moderate inhibitor when IC50 is between 10 $\mu$g/mL and 100 $\mu$g/mL, a weak inhibitor when IC50 is $\geq$ 100 $\mu$g/mL and has no inhibitory activity when IC50 is much > 100 $\mu$g/mL.

The active ingredient used as a comparison for this experiment is a specific inhibitor for the selected isoform and, in particular, Quinidine (CYP2D6 inhibitor). The compound is suspended in DMSO (solvent), at an initial concentration of 13 $\mu$g/mL for Quinidine and then further diluted in deionized water to identify, with the luminometric assay previously described, the IC50 values, i.e. the concentration of active ingredient necessary to reduce the activity of the CYP2D6 isoform to 50%. This value corresponds to 0.0015 $\mu$g/mL of Quinidine for CYP2D6. This assay therefore validates the luminometric method and confirms that the substrate used for the experiment is indeed a potent inhibitor of the selected CYP isoform, compatible with the data present in the literature.

The inhibitory activity of mixture M according to the present invention against CYP2D6 was measured by the luminometric method using a suspension in DMSO of mixture M to obtain a concentration of 100 $\mu$g/mL which is then diluted, after vigorous mechanical shaking, in a wide range of concentrations between 0.25 $\mu$g/mL and 2,500 $\mu$g/mL.

The results obtained demonstrate that at the concentration of mixture M according to the invention of 118.2 $\mu$g/mL the IC50 value for CYP2D6 is detected.

Table 6 reports the effect of exposure to increasing concentrations of mixture M on the activity of the CYP2D6 enzyme, belonging to the cytochrome P450 family (enzyme activity expressed as a percentage compared to the control (vehicle only)). The results are reported as mean $\pm$ standard deviation.

Table 6

| Concentration ($\mu$g/mL) | Activity CYP2D6 (%) |
|---|---|
| 0E+00 | 100.0 $\pm$ 0.4 |
| 2.5E-01 | 100.4 $\pm$ 0.7 |
| 2.5E+00 | 99.6 $\pm$ 1.3 |
| 2.5E+01 | 96.3 $\pm$ 0.5 |
| 1E+02 | 60.9 $\pm$ 2.0 |
| 2.5E+02 | 12.0 $\pm$ 0.5 |
| 1E+03 | 0.1 $\pm$ 0.1 |
| 2.5E+03 | 0.0 $\pm$ 0.0 |

On the basis of these data, it can be stated that referring to the literature classification previously described, the mixture of the invention has only a weak inhibitory activity on CYP2D6 (IC50 > 100 $\mu$g/mL).

The outcome of the studies described therefore demonstrates that the inhibitory capacity of the mixture according to the present invention towards the CYP2D6 isoform is to be considered negligible. This result is particularly positive as it indicates that the potential undesired interactions that could arise from the co-administration of the formulation according to the present invention with other drugs that are metabolized by this type of cytochrome are to be considered equally negligible. For this reason, the mixture of the present invention is also advantageous compared to the traditional active ingredients used in therapy.

The demonstrated non-interference with the activity of cytochrome P450 of the mixtures and compositions of the invention minimizes the risk of pharmacological interactions in which the toxicity can be increased or the therapeutic effects of another drug can be reduced, thus making such mixtures and/or compositions of the invention suitable for use, both as combination therapy and as complementary therapy, as adjuvants to conventionally employed pharmacological treatments.

_In vitro_ study - Antiallergic action of the mixture comprising components (a), (b), (c), (d) and (e)

In order to evaluate the antiallergic action of the mixture according to the invention comprising components (a), (b), (c), (d) and (e), an *in vitro* study was carried out on a rat-derived leukemia cell line (RBL-2H3), widely used as a model simulating mast cells in immunological and allergological studies.

In particular, the antiallergic activity was evaluated through mast cell degranulation by testing the following samples:

- Mixture (boswellia 100 mg + currant 100 mg + helichrysum 50 mg + perilla 200 mg + quercetin 200 mg)
- Boswellia
- Currant
- Helichrysum
- Perilla
- Quercetin

RBL-2H3 cells were pretreated with the test product at three different concentrations, defined following a preliminary cytotoxicity test, and subjected to the degranulation process, by sensitization with anti-DNP IgE (200 ng/ml overnight) and stimulation with DNP-HSA (100 ng/ml for 60 minutes).

At the end of the treatment, the medium was incubated with 4-nitrophenyl-N-acetyl-$\beta$-D-glucosamide and the absorbance measured by spectrophotometric reading.

Degranulation was calculated as the percentage of $\beta$-hexosaminidase (degranulation marker) and the results were compared with those obtained from i) non-degranulating cells (neither sensitized nor stimulated), ii) degranulating cells (sensitized and stimulated, NC) and iii) cells treated with 20 $\mu$M dehydrocostolactone, as a positive control.

## Claims

1. A mixture comprising or, alternatively, consisting of:

   (a) a Boswellia dry extract, preferably a dry extract of the resin, botanical name *Boswellia serrata;* and
   (b) a black currant dry extract, preferably a dry leaf extract, botanical name *Ribes nigrum;* and
   (c) a Helichrysum dry extract, preferably an extract of the flowering tops, botanical name *Helichrysum italicum* and
   (d) a Perilla dry extract, preferably a seed/leaf extract, botanical name *Perilla frutescens*; and
   (e) a quercetin;

   said mixture being for use in a method of treatment, preventive or curative, of an allergic disorder or disease.

2. The mixture for use according to claim 1, wherein said (a) a Boswellia dry extract, preferably a dry extract of the resin of *Boswellia serrata,* comprises one or more of the boswellic acids selected from the group comprising or, alternatively, consisting of: acetyl-11-keto-$\beta$-boswellic acid, alpha-boswellic acid and beta-boswellic acid; more preferably said one or more boswellic acids may be present, in whole or in part, as a phytocomplex, for example a phytocomplex present in a *Boswellia serrata* dry extract.

3. The mixture for use according to claim 1 or 2, wherein said (a) a boswellia dry extract, preferably a dry extract of the resin of *Boswellia serrata,* comprises or, alternatively, consists of:

   - a titre of acetyl-11-keto-$\beta$-boswellic acid NLT (not less than) 10%, preferably comprised from 10% to 50%, more preferably comprised from 10% to 20%, for example 10%, measured by HPLC; and/or;
   - a titre of alpha-boswellic acid preferably comprised from 1% to 20%, more preferably comprised from 2% to 15%, even more preferably comprised from 2% to 15%, measured by HPLC; and/or;
   - a titre of beta-boswellic acid preferably comprised from 5% to 40%, more preferably comprised from 10% to 30%, even more preferably comprised from 19% to 28%, measured by HPLC.

4. The mixture for use according to any of the preceding claims, wherein said (b) blackcurrant dry extract is a dry extract of *Ribes nigrum* leaves, having a rutin titre $\geq$ 4% and preferably comprised from 4% to 15%, more preferably from 4% to 10%; wherein said rutin is present, in whole or in part, as a phytocomplex preferably present in a of *Ribes nigrum* leaves dry extract.

5. The mixture for use according to any of the preceding claims, wherein said (c) a helichrysum dry extract, preferably an extract of the flowering tops *Helichrysum italicum* comprises one or more biologically active compounds such as: essential oils (geraniol, eugenol, neroli, sesquiterpenes, furforol), triterpenes (boswellic acid, ursolic acid), flavonoids (helichrysin, apigenin, quercitrin, narigenin, kaempferol), caffeic acid, phytosterols, tannins, preferably present, in whole or in part, as a phytocomplex present in said extract.

6. The mixture for use according to any of the preceding claims, wherein said (d) perilla dry extract, preferably a dry extract from seeds/leaves of *Perilla frutescens*, has a total polyphenol titre NLT 2.5%, measured by UV, and preferably comprised of from 2.5% to 10%, more preferably from 2.5% to 5%, measured by UV, wherein said polyphenols are preferably present, in whole or in part, as a phytocomplex present in said extract.

7. The mixture for use according to any of the preceding claims, wherein said mixture is for use in a method of treatment, preventive or curative, of a disorder or disease is selected from the group comprising cough, dry cough, angioedema of the upper airways, bronchospasm, asthma, allergic asthma and rhinitis.

8. The mixture for use according to any of the preceding claims, wherein said mixture is for use in a combination therapy or in a complementary therapy, in particular as an adjuvant in a method of treatment, preventive and/or curative and/or symptomatic, anti-allergic of a disorder or disease affecting the respiratory system of an allergic nature, preferably, selected from the group comprising cough, dry cough, angioedema of the upper airways, bronchospasm, asthma, allergic asthma and rhinitis.

9. The mixture for use according to any preceding claim, wherein said mixture further comprises (f) at least one ingredient selected from the group comprising or, alternatively, consisting of a feverfew extract, apple extract, elderberry extract, one or more strains of probiotic bacteria, and/or mixtures thereof.

10. The mixture for use according to any preceding claim, wherein:

- said (f) feverfew dry extract is, preferably, a dry extract of the aerial portion, botanical name *Tanacetum parthenium;*
- said (f) apple extract is, preferably, an extract from immature green apples from Central Asia;
- said (f) elderberry extract is, preferably, a glyceric extract of elderberry;
- said (f) one or more strains of probiotic bacteria are preferably selected from lactobacilli and/or bifidobacteria, more preferably *Lactobacillus acidophilus* SGL11 and *Bifidobacterium animalis ssp. lactis* Bi1 in a quantity, each, of 1 billion CFU to 15 billion CFU.

11. The mixture for use according to any of the preceding claims, wherein:

- said (a) boswellia dry extract, is present in a quantity by weight of 10% to 20%, preferably in a quantity by weight of 12% to 15%, with respect to the total weight of the mixture; and/or
- said (b) blackcurrant dry extract, is present in a quantity by weight of 10% to 20%, preferably in a quantity by weight of 12% to 15%, with respect to the total weight of the mixture; and/or
- said (c) helichrysum dry extract, is present in a quantity by weight of 2% to 10%, preferably in a quantity by weight of 5% to 8%, with respect to the total weight of the mixture; and/or
- said (d) perilla dry extract, is present in a quantity by weight of 20% to 30%, preferably in a quantity by weight of 22% to 28%, with respect to the total weight of the mixture; and/or
- said (e) quercetin, is present in a quantity by weight of 20% to 30%, preferably in a quantity by weight of 22% to 28%, with respect to the total weight of the mixture; and/or
- said (f) feverfew dry extract, is present in a quantity by weight of 10% to 20%, preferably in a quantity by weight of 12% to 15%, with respect to the total weight of the mixture; and/or
- said (f) one or more strains of probiotic bacteria are preferably at least one *Lactobacillus* and at least one *Bifidobacterium,* in a quantity of 1 billion CFU to 15 billion CFU, for each of said strains of bacteria.

12. The mixture for use according to any of the preceding claims, comprising or, alternatively, consisting of:

(a) a boswellia dry extract (i.e. resin) in a quantity of 50 mg to 150 mg, preferably 100 mg; and
(b) a blackcurrant dry extract (i.e. leaves) in a quantity of 50 mg to 150 mg, preferably 100 mg; and
(c) an helichrysum dry extract (i.e. flowering tops) in a quantity of 25 mg to 75 mg, preferably 50 mg; and
(d) a perilla dry extract (i.e. seeds/leaves) in a quantity of 120 mg to 250 mg, preferably 200 mg; and
(e) a quercetin in a quantity of 120 mg to 250 mg, preferably 200 mg; and, optionally,
(f) a feverfew dry extract (i.e. aerial portion) in a quantity of 50 mg to 150 mg; preferably 100 mg; and/or
*Lactobacillus acidophilus* SGL11 and *Bifidobacterium animalis ssp. lactis* Bi1 in a quantity, each, of 1 billion CFU to 15 billion CFU.

13. A composition comprising or, alternatively, consisting of (I) a mixture according to any of claims 1-12, and (II) at least one additive and/or excipient of pharmaceutical or food grade, said composition being for use in a method of treatment, preventive or curative, of an allergic disorder or disease.

14. The composition for use according to claim 13, wherein said composition is formulated preferably for oral administration in a liquid form, preferably solutions, emulsions, suspensions, sprays, and drops; or is formulated preferably for oral administration in a solid form, preferably tablets, capsules, granules and powders.

15. The composition for use according to claim 13 or 14, wherein said disorder or disease is selected from the group comprising cough, dry cough, angioedema of the upper airways, bronchospasm, asthma, allergic asthma and rhinitis.

16. The mixture for use according to claim 15, wherein said composition is for use in a combination therapy or in a complementary therapy, in particular as an adjuvant in a preventive and/or curative and/or symptomatic, anti-allergic method of treatment of a disorder or disease affecting the respiratory system of an allergic nature, preferably, selected from the group comprising cough, dry cough, angioedema of the upper airways, bronchospasm, asthma, allergic asthma and rhinitis.

Europäisches
Patentamt

European
Patent Office

Office européen
des brevets

# EUROPEAN SEARCH REPORT

**Application Number**

EP 25 17 7703

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "DUOALLERGY - Integratore alimentare, favorisce le funzionalità dell'apparato respiratorio", , 21 February 2024 (2024-02-21), XP093225079, Retrieved from the Internet: URL:https://web.archive.org/web/2024022121 1505/https://www.biodemia.it/it/duoallergy * the whole document * ----- | 1-16 | INV. A61K36/185 A61K31/352 A61K35/741 A61K35/747 A61K36/28 A61K36/324 A61K36/35 A61K36/535 A61K36/73 A61P1/00 A61P19/02 A61P29/00 A61P11/06 A61P11/14 A61P37/00 |
| T | Anonymous Home >: "Duoallergy - compressa gastroresistente - integratori per il benessere dell'apparato respiratorio", , 28 October 2024 (2024-10-28), XP093225084, Retrieved from the Internet: URL:https://www.codifa.it/integratori/d/du oallergy-integratori-per-il-benessere-dell -apparato-respiratorio * the whole document * ----- | 1-16 | |
| X | SZELENYI I ET AL: "HERBAL REMEDIES FOR ASTHMA TREATMENT: BETWEEN MYTH AND REALITY", DRUGS OF TODAY / MEDICAMENTOS DE ACTUALIDAD, J.R. PROUS SS.A. INTERNATIONAL PUBLISHERS, ES, vol. 38, no. 4, 1 April 2002 (2002-04-01), pages 265-303, XP008007606, ISSN: 0025-7656, DOI: 10.1358/DOT.2002.38.4.668337 * page 267, left column, first paragraph; page 268, right column, last paragraph bridging with page 269, left column, first paragraph; page 277, left column, third paragraph; page 279, left column, third paragraph. * ----- -/-- | 1-16 | TECHNICAL FIELDS SEARCHED (IPC) A61K A61P |

The present search report has been drawn up for all claims

| Place of search | Date of completion of the search | Examiner |
|---|---|---|
| Munich | 22 August 2025 | Basso, Veronica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................
& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 17 7703

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EJAZ AFAF ET AL: "Biological activities, therapeutic potential, and pharmacological aspects of blackcurrants ( Ribes nigrum L): A comprehensive review", FOOD SCIENCE & NUTRITION, vol. 11, no. 10, 15 August 2023 (2023-08-15), pages 5799-5817, XP093226787, ISSN: 2048-7177, DOI: 10.1002/fsn3.3592 * page 5810, paragraph 6.4 * ----- | 1-16 | |
| A | FURLAN VERONIKA ET AL: "Helichrysum italicum: From Extraction, Distillation, and Encapsulation Techniques to Beneficial Health Effects", FOODS, vol. 12, no. 4, 13 February 2023 (2023-02-13), page 802, XP093226788, CH ISSN: 2304-8158, DOI: 10.3390/foods12040802 * page 1, lines 13-15; page 16 lines 15-17 bridging to page 17, line 1 * ----- | 1-16 | **TECHNICAL FIELDS SEARCHED (IPC)** |

The present search report has been drawn up for all claims

| Place of search | Date of completion of the search | Examiner |
|---|---|---|
| Munich | 22 August 2025 | Basso, Veronica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    .................................................................................

& : member of the same patent family, corresponding document

EPO FORM 1503 03.82 (P04C01)

page 2 of 2

**EP 4 653 006 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 68606-81-5
- *CHEMICAL ABSTRACTS*, 90045-56-0
- *CHEMICAL ABSTRACTS*, 89997-65-9
- *CHEMICAL ABSTRACTS*, 84603-58-7